# EUROPÄISCHE PATENTSCHRIFT

(11) **EP 2 542 934 B1**
(45) Veröffentlichungstag und Bekanntmachung des Hinweises auf die Patenterteilung: **24.08.2016**
(21) Anmeldenummer: 10708920.3
(22) Anmeldetag: 03.03.2010
(51) Int. Cl.: G02B 7/00, A61B 18/20, A61F 9/008

(54) **VORRICHTUNG ZUR GEWICHTSKOMPENSIERENDEN BEWEGLICHEN AUFHÄNGUNG EINES FOKUSSIEROBJEKTIVS EINER LASEREINRICHTUNG**
APPARATUS FOR THE WEIGHT-COMPENSATING MOVABLE SUSPENSION OF A FOCUSSING OBJECTIVE OF A LASER DEVICE
DISPOSITIF DE SUSPENSION MOBILE AVEC COMPENSATION DU POIDS D'UN OBJECTIF DE CONCENTRATION D'UN ÉQUIPEMENT À LASER

(43) Veröffentlichungstag der Anmeldung: 09.01.2013
(73) Patentinhaber: WaveLight GmbH, 91058 Erlangen (DE)
(72) Erfinder: ROBL, Gerhard, 90547 Stein (DE)
(74) Vertreter: Katérle, Axel
(86) Internationale Anmeldenummer: PCT/EP2010/001319
(87) Internationale Veröffentlichungsnummer: WO 2011/107113

(56) Entgegenhaltungen:
- EP-A1- 1 886 639
- WO-A1-2008/098388
- AU-B2- 528 966
- US-A- 5 336 215

## Beschreibung

Die Erfindung befasst sich mit der gewichtskompensierenden beweglichen Aufhängung eines Fokussierobjektivs einer Lasereinrichtung. Die Lasereinrichtung stellt vorzugsweise gepulste Laserstrahlung bereit, die mittels des Fokussierobjektivs auf einen gewünschten Einwirkort der Strahlung fokussiert wird.

Eine gewichtskompensierende bewegliche Aufhängung des Fokussierobjektivs ist nicht nur, jedoch vor allem dort erwünscht, wo es um die Behandlung lebenden Gewebes geht und ein direkter Kontakt des Gewebes mit der Lasereinrichtung besteht. Ein solcher direkter Kontakt wird beispielsweise oftmals bei laserchirurgischen Eingriffen am humanen Auge hergestellt, wenn mit ultrakurzpulsiger Laserstrahlung Schnitte (Inzisionen) in der Kornea oder anderen Augenteilen angebracht werden sollen. Der direkte Kontakt soll dabei eine präzise Positionierung des zu behandelnden Auges relativ zu der Lasereinrichtung in Ausbreitungsrichtung der Strahlung gewährleisten. Üblicherweise wird dem Fokussierobjektiv eine geeignete Schnittstelleneinheit (Patientenadapter) vorgeschaltet, welche die physische Ankopplung des Auges an die Lasereinrichtung bewerkstelligt. Die Schnittstelieneinheit weist in der Regel einen mit dem Fokussierobjektiv gekoppelten Halter für ein mit dem Auge in Kontakt zu bringendes Kontaktelement aus einem für die Laserstrahlung transparenten Material auf. Das Kontaktelement kann beispielsweise eine plane Unterseite zur Einebnung (Applanation) der Kornea aufweisen.

Fokussierobjektive in Lasereinrichtungen sind häufig mehrlinsige Systeme, die ein beträchtliches Gewicht auf die Waage bringen können. Mehrere Kilogramm sind für solche Fokussierobjektive nicht unüblich. Es liegt auf der Hand, dass bei einer Augenoperation nicht das volle Gewicht des Fokussierobjektivs auf dem behandelten Auge liegen darf. Deswegen wird eine gewichtskompensierende Aufhängung für das Fokussierobjektiv vorgesehen. Die Kraft, welche das Fokussierobjektiv bei einer solchen gewichtskompensierenden Lösung noch auf das Auge ausübt, ist z. B auf wenige Newton (z.B. 1 bis 2 N) reduziert. Dies ermöglicht eine sanfte und für das Auge ungefährliche Auslenkung des Fokussierobjektivs nach oben, sollte der Patient unwillkürlich, etwa in einer Panikreaktion, seinen Kopf plötzlich hochheben wollen.

Ein Beispiel einer gewichtskompensierenden Aufhängung eines Fokussierobjektivs findet sich in der US 5,336,215. Dort wird die Gewichtskraft des Objektivs durch ein Federsystem kompensiert, mittels dessen das Objektiv in einem Rahmen relativ zu diesem beweglich aufgehängt ist.

Aufgabe der Erfindung ist es, eine zuverlässig und präzise funktionierende Lösung für die Gewichtskompensation eines Fokussierobjektivs in einer Lasereinrichtung anzugeben.

Die Lösung dieser Aufgabe wird durch die Merkmale von Anspruch 1 definiert. Es ist hierzu eine Vorrichtung zur gewichtskompensierenden beweglichen Aufhängung eines Fokussierobjektivs einer Lasereinrichtung vorgesehen, umfassend:
- eine Krafterzeugungsanordnung zur Erzeugung einer der Gewichtskraft des Fokussierobjektivs entgegenwirkenden Gegenkraftkomponente,
- eine die Gegenkraftkomponente auf das Fokussierobjektiv übertragende, eine Auf/Ab-Ausgleichsbewegung des Fokussierobjektivs gestattende Übertragungsanordnung und
- eine Führungsanordnung zur beweglichen Führung des Fokussierobjektivs, derart, dass bei einer Auf/Ab-Ausgleichsbewegung des Fokussierobjektivs eine optische Achse desselben zumindest ihre räumliche Orientierung und vorzugsweise ihre räumliche Lage beibehält.

Die Führung des Fokussierobjektivs durch die Führungsanordnung verhindert ein unerwünschtes Verkippen der optischen Achse des Objektivs bei einer Auf/Ab-Ausgleichsbewegung desselben. Als Auf/Ab-Ausgleichsbewegung wird hier eine Bewegung des Fokussierobjektivs nach oben bzw. nach unten relativ zur Lasereinrichtung verstanden. Die optische Achse bleibt so trotz einer Ausgleichsbewegung des Objektivs in einer bestimmten Orientierung zum behandelten Objekt, etwa zur optischen Achse eines behandelten humanen Auges. Es ist an dieser Stelle darauf hinzuweisen, dass die Erfindung grundsätzlich bei Lasereinrichtungen für beliebige Anwendungszwecke und insbesondere für die Bearbeitung beliebiger Materialien (auch toter Materie) eingesetzt werden kann und keineswegs auf ophthalmologische Anwendungszwecke beschränkt ist.

Die Krafterzeugungsanordnung umfasst eine Gegengewichtsanordnung, deren Masse zur Erzeugung mindestens eines Teils, insbesondere der gesamten Gegenkraftkomponente dient. Die Gegengewichtsanordnung kann aus einem einzigen Gegengewicht bestehen, sie kann alternativ aus mehreren (mindestens zwei) Einzelgewichten bestehen, die beispielsweise in veränderbarer Anzahl oder/und in veränderbarer relativer Lage zueinander zum Einsatz gebracht werden können. Die Einzelgewichte können auch zu einem einzigen Gesamtgewicht zusammenfügbar sein und einzeln aus dem Gesamtgewicht herausnehmbar sein. Durch solche Variabilitäten der Gegengewichtsanordnung ist eine besonders präzise Tarierung der Gewichtskompensation möglich.

Es soll im Rahmen der Erfindung überdies nicht ausgeschlossen sein, einen Teil der Gegenkraftkomponente mittels wenigstens eines federelastischen Elements zu erzeugen. Wegen des mit federelastischen Elementen häufig verbundenen Nachteils einer Wegabhängigkeit der wirksamen Kraft (die Federkraft hängt vom Verförmungszustand des federelastischen Elements ab) erzeugt die Krafterzeugungsanordnung die Gegenkraftkompönente zweckmäßigerweise nicht ausschließlich mittels federelastischer Elemente. Es ist aber denkbar, ein oder mehrere federelastische Elemente mit einer Gegengewichtsanordnung zu kombinieren, etwa um gezielt eine gewünschte Kraft-Weg-Abhängigkeit zu realisieren.

Die Übertragungsanordnung ist bevorzugt nach Art einer Wippe ausgebildet, auf deren eine Wippenseite die Gegenkraftkomponente einwirkt und auf deren andere Wippenseite die Gewichtskraft des Fokussierobjektivs einwirkt. Man kann die Wippe funktional mit einem Wägebalken vergleichen.

Die Wippe ist beispielsweise von mindestens einem Hebelkörper gebildet, dessen einer Hebelarm mit dem Fokussierobjektiv verbunden ist und dessen anderer Hebelarm mit der Krafterzeugungsanordnung verbunden ist. Es versteht sich, dass zwei oder mehr solcher Hebelkörper im Abstand parallel nebeneinander vorgesehen sein können, um die Wippe zu bilden. Ein Kraftangriffspunkt der Krafterzeugungsanordnung an dem Hebelkörper kann dabei längs desselben verstellbar sein, um so das auf den Hebelkörper wirkende Drehmoment zu justieren. Allgemein gesagt ist vorzugsweise mindestens ein Kraftangriffspunkt der Krafterzeugungsanordnung an der Wippe hinsichtlich seines Abstands von einer Schwenkachse der Wippe justierbar.

Die Verwendung einer an einer Wippenseite (d.h. an einem Hebelarm) angreifenden Gegengewichtsanordnung zur Erzeugung wenigstens eines Teils der Gegehkraftkomponente hat den Vorteil einer weitestgehenden Konstanz der erzeugten Gegenkraftkomponente über den gesamten betriebsmäßig erforderlichen Bewegungshub des Fokussierobjektivs. Durch gezielte Verlagerung des Schwerpunkts der Gegengewichtsanordnung relativ zu der Wippe kann die Kraft-Weg-Kennlinie der Aufhängevorrichtung definiert eingestellt werden. Dies ermöglicht eine Tarierung auch bei einem unteilbaren Gegengewicht. Umfasst die Krafterzeugungsanordnung hingegen wenigstens ein federelastisches Element, so kann durch die Verschiebbarkeit des Kraftangriffspunktes des federelastischen Elements entlang des Hebels bzw. entlang der Wippe zusätzlich zu der federtypischen Kraft-Weg-Charakteristik des federelastischen Elements die Kraft-Weg-Kennlinie der Aufhängevorrichtung eingestellt werden.

Das Fokussierobjektiv ist zweckmäßigerweise an der Wippe relativ zu dieser um eine Drehachse drehbar abgestützt, welche im Abstand parallel zu einer Schwenkachse der Wippe verläuft. Dabei empfiehlt es sich für eine räumliche Lagestabilität der optischen Achse des Objektivs, wenn die Abstützung des Fokussierobjektivs an der Wippe abstandsvariabel zur Schwenkachse der Wippe ist. Bei Kippen der Wippe kann dann das Fokussierobjektiv eine gleichzeitige Bewegung im Sinne einer Abstandsänderung von der Wippenachse ausführen. Das Objektiv kann sich so statt längs einer Kreisbahn längs einer Geraden auf und ab bewegen, d.h. es behält seine transversale Lage (transversal meint orthogonal zur Objektivachse) gegenüber dem bearbeiteten Objekt bei.

Die Schwenklagerung der Wippe ist vorzugsweise reibungsarm gestaltet und kann beispielsweise mittels eines Kunststoff-Gleitlagers oder eines Wälzlagers realisiert sein. Eine hohe Reibungsarmut ist erstrebenswert, um eine Verfälschung der effektiven Kraft-Weg-Charakteristik der Aufhängevorrichtung durch überlagerte Reibungskräfte bestmöglich zu vermeiden.

Das Fokussierobjektiv stützt sich bei einer bevorzugten Ausbildung frei beweglich an einer Stützflächenanordnung der Wippe ab. Insbesondere kann die Stützflächenanordnung so ausgebildet sein, dass das Objektiv lose auf die Wippe aufgelegt werden kann. Die Stützflächenanordnung kann beispielsweise von mindestens einer länglichen Aussparung mindestens eines Hebelkörpers der Wippe gebildet sein, in welche das Objektiv mit einem geeigneten Stützstift oder einer anderen Stützformation eingeführt werden kann. Die Aussparung kann beispielsweise nach oben offen sein, oder/und sie kann - bezogen auf die Längserstreckung des betreffenden Hebelarms, an dem das Objektiv angreift - vorne oder hinten offen sein, so dass die Stützformation des Objektivs von vorne bzw. hinten her in die Aussparung eingeführt werden kann.

Es versteht sich, dass die Stützflächenanordnung alternativ beispielsweise von einer Anordnung eines oder mehrerer ringsum geschlossener Langlöcher gebildet sein, wobei das Objektiv in jedes Langloch mit einem geeigneten Stützvorsprung eingreift. Es versteht sich ferner, dass gemäß einer Abwandlung die Stützflächenanordnung an dem Objektiv und geeignete Stützformationen zur Abstützung an der Stützflächenanordnung an der Wippe ausgebildet sein können.

In jedem Fall ist die Abstützung des Objektivs an der Wippe vorzugsweise derart, dass nicht nur eine Drehung des Objektivs relativ zur Wippe im Zuge einer Schwenkbewegung der Wippe ermöglicht ist sondern auch eine radiale Abstandsänderung zwischen der Drehachse des Objektivs und der Wippenachse (radial bezogen auf die Wippenachse).

Die Führungsanordnung ist als Linearführung mit zur optischen Achse des Fokussierobjektivs paralleler Führungsrichtung ausgebildet. Sie weist vorzugsweise Führungsformationen auf, welche bei einer Auf/Ab-Ausgleichsbewegung des Fokussierobjektivs Bewegungen desselben quer zur optischen Achse unterbinden. Dies dient der Lagestabilisierung des Objektivs im Raum.

Beispielsweise kann die Führungsanordnung ein Linearlager sowie einen darin geführten Schlitten umfassen. Der Schlitten kann dabei an dem Fokussierobjektiv befestigt sein. Das Linearlager kann ferner wenigstens eine Führungsnut umfassen. Der Schlitten kann zudem wenigstens einen Führungsvorsprung umfassen, der dazu eingerichtet ist, mit der Führungsnut derart zusammenzuwirken, dass eine Verschiebung des Schlittens in horizontaler Richtung vermieden wird. Auf diese Weise wird eine Führung des Fokussierobjektivs in vertikaler Richtung ermöglicht, bei der die optische Achse des Fokussierobjektivs bei einer Ausgleichsbewegung desselben gegenüber einer Ausgangsstellung ihre Ausrichtung und Lage beibehalten kann. Die Führungsnut kann hierzu mit einem Hinterschnitt ausgebildet sein, in den der Führungsvorsprung eingreift.

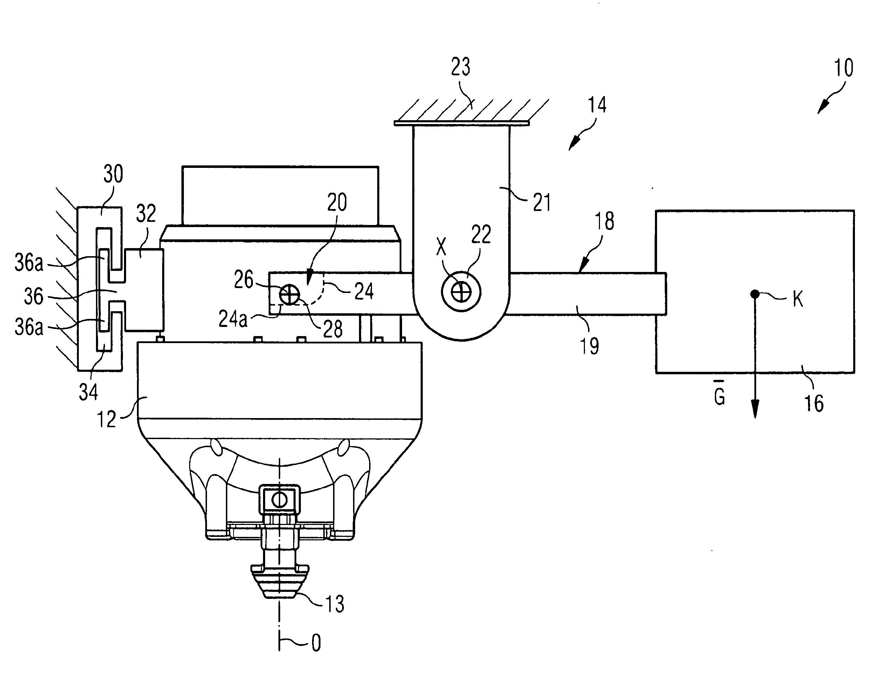

Die Erfindung wird nachfolgend anhand der beigefügten Zeichnung weiter erläutert. Deren Figur 1 zeigt schematisch und in keiner Weise maßstabsrealistisch ein Ausführungsbeispiel einer Lasereinrichtung zur Anbringung von Schnitten in der Hornhaut oder anderen Gewebeteilen eines humanen Auges.

Von der allgemein mit 10 bezeichneten Lasereinrichtung ist nur die "Endstufe" zeichnerisch dargestellt, nämlich im wesentlichen nur ein Fokussierobjektiv 12 mit einem strahlungsaustrittsseitig daran angeordneten Patientenadapter 13. Das Fokussierobjektiv 12 ist durch eine Aufhängevorrichtung 14 gewichtskompensiert und beweglich aufgehängt. Wenn hier von einer Gewichtskompensation die Rede ist, so meint dies eine Kompensation zumindest eines überwiegenden Teils des Gewichts des Objektivs 12. Ein insbesondere kleiner Restteil des Objektivgewichts kann unkompensiert bleiben, beispielsweise in der Größenordnung von einigen wenigen Newton Gewichtskraft. Das Fokussierobjektiv 12 fokussiert in nicht näher dargestellter Weise ein afokales Strahlenbündel der von der Lasereinrichtung 10 bereitgestellten Laserstrahlung auf einen Fokusort innerhalb des zu schneidenden Augengewebes. Die verwendete Laserstrahlung besitzt beispielsweise Pulsdauern im Bereich von Femtosekunden und eine Wellenlänge im niederen Infrarotbereich (etwa zwischen ca. 1000 und ca. 1100 nm) oder im UV-Bereich, vorzugsweise oberhalb etwa 300 nm.

Die Aufhängevorrichtung 14 weist in der dargestellten Ausführungsform der Figur 1 eine Krafterzeugungsanordnung mit einem Gegengewicht 16 auf, das eine vertikal gerichtete Gewichtskraftkomponente G erzeugt, mit der die Gewichtskraft des Fokussierobjektivs 12 zumindest teilweise kompensiert werden soll. Ferner umfasst die Aufhängevorrichtung 14 eine Übertragungsanordnung in Form einer Wippe 18, welche an ihrem in Figur 1 rechten Wippenende mit dem Gegengewicht 16 lösbar verbunden ist, während sie an ihrem anderen, in Figur 1 linken Ende eine Lageraufnahme 20 zur lösbaren Kopplung mit dem Fokussierobjektivs 12 aufweist. Das Gegengewicht 16 kann entlang des betreffenden Hebelarms der Wippe verschoben werden, wodurch der effektive Kraftangriffspunkt K des Gegengewichts und somit das wirksame Gegenkraftmoment verändert werden kann.

Die Wippe 18 ist mittels eines Lagers 22 um eine im Abstand zwischen den Angriffspunkten des Objektivs 12 und des Gegengewichts 16 liegende Schwenkachse X drehbar gelagert. Das Lager 22 bietet eine Abstützung der Wippe 18 über eine oder mehrere Verbindungswangen 21 an einem schematisch angedeuteten Tragteil 23, das seinerseits stationär angeordnet sein kann oder relativ zu anderen Komponenten der Lasereinrichtung 10 beweglich angeordnet sein kann.

Die Wippe 18 kann beispielsweise eine längliche, insbesondere im wesentlichen gerade Hebelstange 19 aufweisen, die an einem ihrer Stangenenden mit dem Objektiv 12 und an dem anderen Stangenende mit dem Gegengewicht 16 gekoppelt ist. Zweckmäßigerweise sind zwei solcher Hebelstangen 19 beidseits des Objektivs 12 (d.h. in der Blickrichtung der Figur 1 vor und hinter dem Objektiv 12) parallel zueinander vorgesehen. Im Verlauf der weiteren Beschreibung wird jedoch stets nur von einer Hebelstange die Rede sein. Die nachfolgenden Ausführungen gelten jedoch sinngemäß für beide Hebelstangen.

Die erwähnte Lageraufnahme 20 ist im gezeigten Beispielfall von einer seitlich angebrachten Aussparung 24 der Hebelstange 19 gebildet (gestrichelt angedeutet), welche mehrseitig offen ist, nämlich nach oben sowie in der Figur nach links. Der Boden 24a dieser Aussparung 24 bildet eine Stützfläche für einen von dem Objektiv 12 seitlich abstehenden, in die Aussparung hineinragenden Stützzapfen 26. Das Fokussierobjektiv 12 liegt über seinen Stützzapfen 26 lose auf der Hebelstange 19 auf. Bei einer Verschwenkung der Wippe 18 erfolgt nicht nur eine Drehung des Objektivs 12 gegenüber der Wippe 18 um die Zapfenachse sondern auch eine Verlagerung des Stützzapfens 26 innerhalb der Lageraufnahme 20. Bei dieser Verlagerung andert sich der radiale Abstand des Stützzapfens 26 von der Schwenkachse X. Dies ermöglicht es, trotz einer Auf- oder Abbewegung des Objektiv 12 die räumliche Lage und Orientierung einer optischen Objektivachse O unverändert zu halten. Um die Verlagerung des Stützzapfens 26 in der Lageraufnahme 20 möglichst reibungsarm zu gestalten, kann der Stützzapfen 26 beispielsweise einen gleit- oder wälzgelagerten Ring 28 tragen, welcher am Boden 24a der Aussparung 24 abrollen kann.

Die Aufhängevorrichtung 14 umfasst des weiteren eine Führungsanordnung zur vertikalen Linearführung des Fokussierobjektivs 12. Während die Verlagerbarkeit des Stützzapfens 26 in der Lageraufnahme 20 die Voraussetzung für eine gleichbleibenden räumliche Lage der Objektivachse 0 bei einer Bewegung des Objektivs 12 schafft, gewährleistet die Führungsanordnung, dass die Objektivachse O tatsächlich nicht verkippt oder transversal (quer zur Achse O) versetzt wird.

Die Führungsanordnung umfasst in der dargestellten Ausführungsform ein gegenüber dem Fokussierobjektiv 12 feststehendes Linearlager 30 sowie einen daran geführten Schlitten 32, der an dem Fokussierobjektiv 12 befestigt ist. Das Linearlager 30 ist mit einer Führungsnut 34 versehen, die in der Art eines Hinterschnitts ausgebildet ist und in die ein Führungsvorsprung 36 des Schlittens 32 eingreift. Der Führungsvorsprung 36 ist in der in Figur 1 gezeigten Ausführungsform im Querschnitt T-förmig ausgebildet, wobei die beiden T-Seitenschenkel 36a des Führungsvorsprungs 36 in die hinterschnittene Führungsnut 34 derart eingreifen, dass eine horizontale (transversale) Bewegung des Fokussierobjektivs 12 von dem Linearlager 30 weg (in Figur 1 nach rechts) verhindert ist. Zudem wird durch den Eingriff der T-Seitenschenkel 36a in die hinterschnittene Führungsnut 34 gewährleistet, dass das Fokussierobjektiv 12 nicht verkippt. Es wird durch die Führungsanordnung stets in einer bestimmten Orientierung gehalten.

Der Eingriff zwischen dem Führungsvorsprung 36 und der Führungsnut 34 bietet ausreichend vertikales Bewegungsspiel, um den nötigen Bewegungshub des Fokussierobjektivs 12 in vertikaler Richtung zu gewährleisten.

Wird das Fokussierobjektiv 12 mit seinem Patientenadapter 13 auf das zu behandelnde Auge (nicht dargestellt) aufgesetzt, so kann das Fokussierobjektiv 12 durch einen von dem Auge bewirkten geringfügigen Gegendruck in vertikaler Richtung nach oben auslenken. Der Stützzapfen 26 verlagert sich bei dieser Ausgleichsbewegung des Fokussierobjektivs 12 rollend (oder alternativ gleitend) entlang des Bodens 24a der Aussparung 24, während sich die Wippe 18 im Uhrzeigersinn um die Wippenachse X dreht und sich gleichzeitig das Objektiv 12 relativ zur Wippe 18 um die Achse des Stützzapfens 26 dreht. Wäre dagegen in einem gedachten hypothetischen Fall das Fokussierobjektiv 12 starr mit der Wippe 18 verbunden, würde eine Auf- oder Abwärtsbewegung des Fokussierobjektivs 12 zu einer Verkippung der optischen Achse O führen. Ein solches Verkippen ist jedoch im gezeigten Ausführungsbeispiel aufgrund der beweglichen Abstützung des Objektivs 12 an der Wippe 18 und aufgrund der linearen Führung des Objektivs 12 durch das Linearlager 30 ausgeschlossen.

## Patentansprüche

1. Lasereinrichtung (10), umfassend:
- eine Quelle für Laserstrahlung,
- ein Fokussierobjektiv (12) zur Fokussierung der Laserstrahlung, wobei das Fokussierobjektiv eine optische Achse (O) aufweist,
- eine strahlungsaustrittsseitig an dem Fokussierobjektiv angeordnete Schnittstelleneinheit mit einem für die Laserstrahlung transparenten Kontaktelement zur Anlage an einem mit der Laserstrahlung zu bearbeitenden Objekt,
- eine Vorrichtung (14) zur gewichtskompensierenden beweglichen Aufhängung des Fokussierobjektivs, **dadurch gekennzeichnet, dass** diese Vorrichtung eine um eine Wippenachse (X) schwenkbar an einer Gehäusekomponente der Lasereinrichtung abgestützte Wippe (18) mit zwei Wippenarmen aufweist, wobei an einem ersten der Wippenarme das Fokussierobjektiv relativ zur Wippe um eine zur Wippenachse parallele Drehachse drehbar abgestützt ist und an dem anderen, zweiten Wippenarm eine Krafterzeugungsanordnung zur Erzeugung einer der Gewichtskraft des Fokussierobjektivs entgegenwirkenden Gegenkraftkomponente angreift, wobei die Krafterzeugungsanordnung eine Gegengewichtsanordnung (16) umfasst, deren Masse zur Kompensation zumindest eines überwiegenden Teils der Gewichtskraft des Fokussierobjektivs dient, wobei die Vorrichtung ferner eine Führungsanordnung (30, 32) zur linearbeweglichen Führung des Fokussierobjektivs längs der optischen Achse (O) desselben aufweist.

2. Lasereinrichtung nach Anspruch 1, wobei die Laserstrahlung gepulst ist.

3. Lasereinrichtung nach Anspruch 1 oder 2, wobei die Schnittstelleneinheit (13) lösbar mit dem Fokussierobjektiv (12) gekoppelt ist.

4. Lasereinrichtung nach einem der vorhergehenden Ansprüche, wobei die Führungsanordnung (30, 32) dazu eingerichtet ist, dass bei einer Auf/Ab-Ausgleichsbewegung des Fokussierobjektivs (12) die optische Achse (O) desselben ihre räumliche Orientierung und ihre räumliche Lage beibehält.

5. Lasereinrichtung nach einem der vorhergehenden Ansprüche, wobei mindestens ein Kraftangriffspunkt (K) der Krafterzeugungsanordnung an dem zweiten Wippenarm hinsichtlich seines Abstands von der Wippenachse (X) justierbar ist.

6. Lasereinrichtung nach einem der vorhergehenden Ansprüche, wobei die Krafterzeugungsanordnung zusätzlich zur Gegengewichtsanordnung (16) wenigstens ein federelastisches Element umfasst.

7. Lasereinrichtung nach einem der vorhergehenden Ansprüche, wobei die Gegengewichtsanordnung (16) aus einem einzigen Gegengewicht besteht.

8. Lasereinrichtung nach einem der Ansprüche 1 bis 6, wobei die Gegengewichtsanordnung aus mehreren Einzelgewichten besteht.

9. Lasereinrichtung nach Anspruch 8, wobei die mehreren Einzelgewichte in veränderbarer Anzahl oder/und in veränderbarer relativer Lage zueinander an dem zweiten Wippenarm anbringbar sind.

10. Lasereinrichtung nach Anspruch 8, wobei die Einzelgewichte zu einem einzigen Gesamtgewicht zusammenfügbar sind.

11. Lasereinrichtung nach einem der vorhergehenden Ansprüche, wobei die Abstützung des Fokussierobjektivs (12) an der Wippe (18) abstandsvariabel zur Wippenachse (X) ist.

12. Lasereinrichtung nach einem der vorhergehenden Ansprüche, wobei die Führungsanordnung (30, 32) Führungsformationen (34, 36) aufweist, welche bei einer Auf/Ab-Ausgleichsbewegung des Fokussierobjektivs (12) Bewegungen desselben quer zur optischen Achse (O) unterbinden.

## Claims

1. A laser system (10), comprising:
- a source for laser radiation,
- a focussing objective (12) for focussing the laser radiation, wherein the focussing objective has an optical axis (0),
- an interface unit which is arranged on the radiation exit side of the focussing objective with a contact element which is transparent for the laser radiation, for placing on an object on which work is to be done using the laser radiation,
- an apparatus (14) for the weight-compensating movable suspension of the focussing objective, **characterised in that** this apparatus comprises a rocker (18) with two rocker arms, which is supported on a housing member of the laser system and swivel-mounted about a rocker axis (X), wherein the focussing objective is rotatably supported relative to the rocker about an axis of rotation which extends parallel to the rocker axis on a first one of the rocker arms, and a force generation device engages with the other second rocker arm for generating a counterforce component which counteracts the weight of the focussing objective, wherein the force generation device comprises a counterweight arrangement (16) whose mass compensates for at least a part of the weight of the focussing objective, and wherein the apparatus also has a guidance system (30, 32) for the linear movable guidance of the focussing objective along the optical axis (0) thereof.

2. The laser system according to Claim 1, wherein the laser radiation is pulsed.

3. The laser system according to Claim 1 or 2, wherein the interface unit (13) is separably coupled with the focussing objective (12).

4. The laser system according to one of the previous claims, wherein the guidance system (30, 32) is configured in such a manner that during an upward/ downward compensatory movement of the focussing objective (12) the optical axis (0) of the focussing objective maintains its orientation in space and its position in space.

5. The laser system according to one of the previous claims, wherein at least one force application point (K) of the force generation device on the second rocker arm may be adjusted with respect to its distance from the swivelling axis (X) of the rocker.

6. The laser system according to one of the previous claims, wherein the force generation device comprises at least one resilient element in addition to the counterweight arrangement (16).

7. The laser system according to one of the previous claims, wherein the counterweight arrangement (16) consists of a single counterweight.

8. The laser system according to one of Claims 1 to 6, wherein the counterweight arrangement (16) consists of several individual weights.

9. The laser system according to Claim 8, wherein the several individual weights may be mounted on the second rocker arm in a variable number and/or in variable relative positions to each other.

10. The laser system according to Claim 8, wherein the several individual weights may be combined to a single total weight.

11. The laser system according to one of the previous claims, wherein the support of the focussing objective (12) on the rocker (18) is at a variable distance to the swivelling axis (X) of the rocker.

12. The laser system according to one of the previous claims, wherein the guidance system (30, 32) comprises guidance formations which prevent movements of the focussing objective (12) transverse to the optical axis (0) in the case of an upward/ downward compensatory movement of the focussing objective.

## Revendications

1. Equipement à laser (10), comprenant :
- une source de rayonnement laser,
- un objectif de concentration (12) prévu pour focaliser le rayonnement laser, cet objectif de concentration présentant un axe optique (0),
- une unité d'interface disposée côté sortie du rayonnement sur l'objectif de concentration et pourvue d'un élément de contact transparent au rayonnement laser destiné à venir en butée contre un objet à traiter par rayonnement laser,
- un dispositif (14) prévu pour assurer la suspension mobile de l'objectif de concentration avec compensation du poids de ce dernier, **caractérisé en ce que** ce dispositif présente une bascule (18) pourvue de deux bras de bascule et prenant appui de façon pivotante autour d'un axe de bascule (X) sur un élément de boîtier de l'équipement à laser, l'objectif de concentration prenant appui de manière pivotante autour d'un axe de pivotement parallèle à l'axe de bascule, par rapport à la bascule, sur un premier desdits bras de bascule, et un ensemble générateur de force destiné à générer une composante de force contraire qui vient contrer le poids de l'objectif de concentration étant en prise sur l'autre deuxième bras de bascule, cet ensemble générateur de force comportant un ensemble formant contrepoids (16) dont la masse sert à compenser au moins une partie majeure de la force de pesanteur de l'objectif de concentration, le dispositif présentant en outre un système de guidage (30, 32) destiné au guidage mobile linéaire de l'objectif de concentration le long de l'axe optique (0) de celui-ci.

2. Equipement à laser (10) selon la revendication 1, le rayonnement laser étant pulsé.

3. Equipement à laser (10) selon la revendication 1 ou 2, l'unité d'interface (13) étant accouplée de manière amovible à l'objectif de concentration (12).

4. Equipement à laser (10) selon l'une des revendications précédentes, l'ensemble de guidage (30, 32) étant conçu pour que lors d'un mouvement de compensation vers le haut/bas de l'objectif de concentration (12), l'axe optique (0) de celui-ci conserve son orientation et sa position dans l'espace.

5. Equipement à laser (10) selon l'une des revendications précédentes, au moins un point d'application de force (K) de l'ensemble générateur de force sur le deuxième bras de bascule pouvant être ajusté quant à sa distance par rapport à l'axe de bascule (X).

6. Equipement à laser (10) selon l'une des revendications précédentes, l'ensemble générateur de force comprenant en plus de l'ensemble formant contrepoids (16) au moins un élément formant ressort.

7. Equipement à laser (10) selon l'une des revendications précédentes, l'ensemble formant contrepoids (16) étant constitué d'un seul contrepoids.

8. Equipement à laser (10) selon l'une des revendications 1 à 6, l'ensemble formant contrepoids étant constitué de multiples poids individuels.

9. Equipement à laser (10) selon la revendication 8, les multiples poids individuels pouvant être placés en nombre variable ou/et dans une position relative variable les uns par rapport aux autres sur le deuxième bras de bascule.

10. Equipement à laser (10) selon la revendication 8, les poids individuels pouvant être assemblés pour former un seul poids total.

11. Equipement à laser (10) selon l'une des revendications précédentes, l'appui de l'objectif de concentration (12) sur la bascule (18) pouvant s'effectuer selon une distance variable par rapport à l'axe de bascule (X).

12. Equipement à laser (10) selon l'une des revendications précédentes, l'ensemble de guidage (30, 32) présentant des formations de guidage (34, 36) lesquelles, lors d'un mouvement de compensation vers le haut/bas de l'objectif de concentration (12), empêchent à celui-ci de se déplacer perpendiculairement à l'axe optique (O).
